Europäisches Patentamt

(19) European Patent Office

Office européen des brevets

(11) Numéro de publication : **0 478 630 B1**

(12) **FASCICULE DE BREVET EUROPEEN**

(45) Date de publication du fascicule du brevet :
01.09.93 Bulletin 93/35

(51) Int. Cl.⁵ : **C12Q 1/68, G01N 33/531**

(21) Numéro de dépôt : **90909455.9**

(22) Date de dépôt : **12.06.90**

(86) Numéro de dépôt international :
**PCT/FR90/00410**

(87) Numéro de publication internationale :
**WO 90/15881 27.12.90 Gazette 90/29**

(54) PROCEDE DE DETECTION DE SEQUENCES SPECIFIQUES D'ACIDES NUCLEIQUES ET SES APPLICATIONS.

(30) Priorité : **12.06.89 FR 8907710**
**08.08.89 FR 8910643**

(43) Date de publication de la demande :
**08.04.92 Bulletin 92/15**

(45) Mention de la délivrance du brevet :
**01.09.93 Bulletin 93/35**

(84) Etats contractants désignés :
**AT BE CH DE DK ES FR GB IT LI LU NL SE**

(56) Documents cités :
**EP-A- 231 495**
**EP-A- 297 379**
**EP-A- 0 310 229**
**EP-A- 0 328 829**
**WO-A-84/01174**
**GB-A- 2 202 328**
**US-A- 4 257 774**
**US-A- 4 824 776**

(56) Documents cités :
**Nucleic Acids Research, vol. 17, no. 7, avril 1989, IRL Press, H. Voss et al.: "Direct genomic fluorescent on-line sequencing and analysis using in vitro amplification of DNA", pp. 2517-2527**
**Proc. Natl. Acad. Sci. USA, vol. 85, janvier 1988, D.R. Engelke et al.: "Direct sequencing of enzymaticallly amplified human genomic DNA", pp. 544-558, voir l'article en entier, en particlier pp. 545-546**

(73) Titulaire : **CIS BIO INTERNATIONAL**
**RN 306**
**F-91400 Saclay (FR)**

(72) Inventeur : **TEOULE, Robert**
**52, rue Thiers**
**F-38000 Grenoble (FR)**
Inventeur : **LIVACHE, Thierry**
**20, rue Claude-Kogan**
**F-38000 Grenoble (FR)**
Inventeur : **FOUQUE, Brigitte**
**3 avenue Aristide Bergès**
**F-38170 Seyssinet (FR)**
Inventeur : **SAUVAIGO, Sylvie**
**28, rue Emile-Gueymard**
**F-38000 Grenoble (FR)**

(74) Mandataire : **Ores, Irène et al**
**CABINET ORES 6, Avenue de Messine**
**F-75008 Paris (FR)**

## Description

La présente invention est relative à un procédé de détection de faibles quantités d'acides nucléiques obtenus par amplification, ainsi qu'à ses applications.

L'une des premières méthodes d'amplification décrites, est la réaction de polymérisation en chaîne (PCR), développée par SAIKI R. et al. (Science, 1985, 230, 1350). Cette technique permet notamment d'amplifier des séquences d'ADN double brin et est basée sur le fonctionnement cyclique d'une ADN polymérase, laquelle enzyme est capable de copier un brin d'ADN, utilisé comme matrice, en un brin complémentaire par élongation à partir de l'extrémité 3' OH libre d'une amorce oligonucléotidique.

La technique PCR consiste à effectuer n cycles successifs d'amplification, au cours desquels deux amorces dirigent l'amplification de la séquence d'ADN double brin qu'elles encadrent.

Un cycle d'amplification est composé de trois étapes permettant de réaliser successivement la dénaturation de l'ADN (95°C), l'hybridation des amorces (37-55°C) et l'extension des brins d'ADN par une ADN polymérase.

Les bases technologiques de la PCR reposent sur deux points fondamentaux :
- l'utilisation de deux amorces oligonucléotidiques , l'une complémentaire du brin ADN+, l'autre du brin ADN- et dont les extrémités 3' sont en regard ;
- l'utilisation répétitive de l'activité d'une ADN polymérase appropriée.

La PCR permet alors l'utilisation, dans des conditions adéquates, de toutes les séquences néosynthétisées au cycle n, comme matrices pour l'ADN polymérase au cycle (n+1). Il en résulte une amplification exponentielle du nombre de séquences d'acides nucléiques cibles, en fonction du nombre de cycles, conformément à la courbe d'amplification qui comprend une phase exponentielle où la quantité Q de séquences cibles obtenues peut être reliée à la quantité Qo de séquences cibles initiales, au facteur d'amplification x et au nombre de cycles n, par la formule suivante : $Q = Qo (1 + x)^n$.

La PCR permet ainsi d'amplifier, de manière exponentielle, des séquences d'acides nucléiques dites cibles, des milliers de fois.

Cette technique d'amplification est plus particulièrement décrite dans la Demande de Brevet européen CETUS 201 184, qui spécifie notamment que les deux amorces oligonucléotidiques mises en oeuvre dans la PCR sont de préférence simple brin et doivent être suffisamment longues pour amorcer la synthèse du produit d'extension en présence de l'ADN polymérase.

Un certain nombre d'améliorations ou de perfectionnements à cette technique ont été proposés et notamment, pour éviter l'addition d'enzymes à chaque cycle, une ADN polymérase pouvant résister à 100°C, la Taq polymérase, décrite dans la Demande de Brevet européen CETUS 258 017, est ajoutée dès le début du procédé et permet de réaliser un nombre important de cycles d'amplification consécutifs. La Taq polymérase a permis d'une part d'automatiser le procédé d'amplification et d'autre part d'augmenter la spécificité de l'amplification.

La PCR permet ainsi d'obtenir, sans clonage, une amplification considérable d'une séquence nucléique, dite séquence cible, donc un énorme gain en sensibilité. La séquence cible amplifiée est ensuite directement accessible à différents procédés d'analyse tel que le procédé de dot-blot, l'électrophorèse ou le séquençage. Deux Demandes de Brevet (Demandes de Brevet européen CETUS n° 200 362 et n° 229 701) décrivent plus particulièrement l'association amplification-hybridation pour la détection d'une séquence d'acide nucléique recherchée dans un échantillon.

Depuis, des variantes et/ou des perfectionnements, pour rendre l'étape ,de détection des séquences amplifiées plus aisée, ont été proposés et concernent notamment les amorces et/ou les sondes utilisées.

L'un de ces perfectionnements est l'utilisation de deux sondes différentes dont l'une, marquée, est dite sonde de détection et. dont l'autre comprend un fragment ayant une affinité pour un autre composant et est dite sonde de capture [Brevet britannique 2 169 403, Brevet français 85 19394, KOHNE D. (American Clin. Review, nov. 1986), Brevet US 4 486 539, Demandes de Brevet européen 70685 et 70687].

En variante, ce sont les amorces elles-mêmes qui sont modifiées, de manière à permettre la capture de l'hybride formé (Demande de Brevet français 88 03107, Demande de Brevet européen MOLECULAR DIAGNOSIS Inc., n° 297 379).

Un autre de ces perfectionnements est illustré par la méthode décrite dans la Demande de Brevet européen Syntex, n° 300 796, qui propose une variante de la PCR qui augmente la sensibilité de cette dernière, notamment en présence de quantités très faibles d'acide nucléique sans en augmenter toutefois la spécificité.

Cependant, l'utilisation en routine de la PCR, a fait apparaître un inconvénient important, lié à son extrême sensibilité, à savoir l'émergence de faux-positifs, qui se sont révélés être liés, après une analyse soigneuse des résultats, essentiellement à une contamination de l'échantillon à analyser par des séquences homologues.

Un certain nombre d'articles, parus depuis environ deux ans, décrivent de tels cas de contaminations et

EP 0 478 630 B1

proposent des moyens pour les résoudre :

- Y.M.D. LO et al., dans un article paru dans Lancet (1988, ii, 679), font apparaître cet inconvénient de la réaction de polymérisation en chaîne, dû à sa sensibilité et, pour pallier cet inconvénient important, préconisent de prendre un soin particulier dans la préparation de la matrice d'ADN avant l'amplification.
- R.A. GIBBS et al., dans un article paru dans Genes & Development, 1989, 3, 1095-1098, prodiguent les conseils suivants : isoler tous les réactifs utilisés pour la mise en oeuvre de la PCR, des équipements utilisés pour analyser les produits obtenus ; utiliser des bouchons à vis et même aller jusqu'à congeler les contenus, avant d'effectuer la réaction finale, afin d'éviter la formation d'aérosols ; limiter le nombre de cycles d'amplification au minimum, à la fois pour réduire le risque d'amplifier de petites quantités de contaminants déjà présents et pour réduire la quantité totale de produits de réaction et disposer de jeux de pipettes différents pour chaque tâche.
- G. SARKAR et al., dans un article paru dans Nature, 1990, **343**, 27, proposent, pour éviter la contamination, de traiter l'échantillon aux rayons UV, avant d'ajouter la matrice d'ADN à amplifier.
- S. KWOK et al., dans un article paru dans Nature, 1989, 339, 237-238, proposent un certain nombre de "bonnes pratiques de laboratoire" pour contrôler et pour éviter la contamination : isoler physiquement les préparations et les produits pour PCR (pièces séparées, rayons UV...) ; autoclaver les tampons utilisés ; diviser les réactifs en petits échantillons afin d'éviter un pipetage répété ; utiliser des gants de protection ; éviter les projections ; utiliser des pipettes ne provoquant l'apparition d'aérosols, etc...

Cependant, les suggestions proposées par les différents Auteurs précités, pour éviter la contamination, n'envisagent en aucun cas le problème des faux-positifs dû à l'amplification de séquences hétérologues et/ou à la présence de débris cellulaires.

De telles séquences hétérologues, différentes des séquences cibles à détecter, mais cependant de structures suffisamment proches pour entraîner une hybridation imparfaite entre les amorces spécifiques des séquences cibles à détecter et ces séquences hétérologues, sont amplifiées de manière exponentielle comme les séquences cibles, lors de la PCR.

En effet, aucune des méthodes d'amplification et/ou de détection de séquences spécifiques d'acides nucléiques de l'Art antérieur, qui sont les seules à être aisément automatisables, ne permet :

1. d'éliminer les séquences hétérologues amplifiées exponentiellement lors de la PCR, conséquence directe de l'intégration physique des amorces dans les brins néosynthétisés, l'amplification de ces séquences hétérologues conduisant à l'émergence de faux positifs ; et/ou

2. de réaliser une détection d'acide nucléique en phase homogène, c'est-à-dire ne nécessitant ni séparation sur gel d'électrophorèse, ni fixation sur un support solide de capture, ni utilisation d'amorces nucléotidiques modifiées, ni une étape d'hybridation avec une sonde de détection.

C'est pourquoi, la Demanderesse s'est en conséquence donné pour but de pourvoir à un procédé de détection tant qualitatif, semi-quantitatif que quantitatif et d'identification de faibles quantités d'acides nucléiques par amplification, notamment en phase homogène, qui répond mieux aux nécessités de la pratique que les procédés de l'Art antérieur, notamment en ce qu'il est facile à automatiser, spécifique, sensible, rapide et économique et en ce que la présence de séquences hétérologues et/ou de débris cellulaires, n'a pratiquement pas d'influence sur ladite détection.

La présente invention a pour objet un procédé de détection de séquences spécifiques d'acide(s) nucléique(s) (séquence unique et/ou mélange de séquences d'acides nucléique) présente(s) dans un échantillon biologique, comprenant au moins une amplification enzymatique, caractérisé en ce qu'après mise en solution appropriée de l'échantillon biologique pour extraire le/les acides nucléiques, ledit procédé comprend la détection d'une séquence d'acide nucléique ou d'un mélange de séquences à l'aide des étapes suivantes :

(1) une étape d'enrichissement en séquence(s) cibles(s) par :

(a) mise en contact de l'échantillon biologique mis en solution, avec au moins une paire d'amorces appropriées, pour amplifier au moins un fragment de ladite/desdites séquences d'acide nucléique cible, lesdites amorces s'hybridant à ladite/lesdites séquences cibles et permettant d'obtenir une solution d'amplification d'enrichissement ; et

(b) au moins une dilution, comprise entre 1/50e et 1/100 000e, de la solution d'amplification d'enrichissement obtenue en (a) ;

(2) une étape de détection des séquences cibles amplifiées obtenues, par :

(c) mise en contact d'une fraction de la solution d'enrichissement obtenue en (b) avec au moins une paire d'amorces dont au moins l'une des séquences est incluse dans la séquence cible amplifiée en (a) pour amplifier au moins un fragment de ladite/desdites séquences d'acide nucléique cible ; et

(d) détection des copies d'acide nucléique cible double brin obtenues en (c), par tout moyen approprié.

On désignera ci-après cette dernière amplification sous le nom de "deuxième série d'amplifications".

Conformément à l'invention, les étapes (a) et (b) sont répétées au moins une fois.

Selon une disposition avantageuse de ce mode de mise en oeuvre, la dilution de l'étape (b) est comprise entre 1/50e et 1/50 000e.

Selon une modalité avantageuse de cette disposition, la dilution de l'étape (b) est comprise entre 1/50e et 1/10 000e, de préférence entre 1/200e et 1/5 000e.

Cette dilution permet d'initialiser l'amplification de détection à partir d'un matériel biologique purifié et ayant les propriétés suivantes :
- il est, d'un part, enrichi en séquences cibles et
- il est, d'autre part, appauvri en séquences "parasites" indésirées, à savoir en amorces non utilisées de l'étape (a), en séquences hétérologues, en acide nucléique total par rapport à l'échantillon biologique initial et en débris cellulaires.

Le fait que les amorces non utilisées de l'étape (a) sont diluées, a l'avantage d'éviter une compétition entre les amorces de l'étape (a) et les amorces de l'étape (c), au moment de la deuxième série d'amplifications. De plus, ceci permet d'utiliser moins d'amorces lors de la deuxième série d'amplifications et d'obtenir un meilleur rendement d'incorporation de ces dernières. Ceci présente un avantage important, car les amorces de l'amplification de détection sont généralement coûteuses et plus difficiles à synthétiser.

Cet enrichissement en séquences cibles par dilution de la solution d'amplification de l'étape (a) permet de mettre les séquences hétérologues qui provoquent des faux démarrages de lecture par hybridation fautive et les débris cellulaires, dans des conditions peu propices à leur amplification lors de la deuxième série d'amplifications et lors de la détection.

Le procédé conforme à l'invention a notamment pour avantage d'augmenter la, spécificité du dosage en abaissant la quantité d'acide nucléique résiduaire et des séquences hétérologues diverses provenant du prélèvement biologique.

Conformément à l'invention, les amorces de l'étape (a) sont des séquences nucléotidiques simple brin (amorce simple (As)), qui s'hybrident à une séquence cible.

De telles amorces peuvent éventuellement être modifiées. Ceci permet d'obtenir le cas échéant, soit une amorce de capture, soit une amorce de détection.

Egalement conformément à l'invention, les amorces de l'étape (c) sont des paires choisies dans le groupe qui comprend les paires : amorce modifiée par un système de capture-amorce modifiée par un système de détection (Ac-Ad), les paires amorce simple-amorce simple (As1-As2), les paires amorce simple-amorce modifiée par un système de capture (As1-Ac), les paires amorce simple-amorce modifiée par un système de détection (As1-Ad).

On entend par amorce de capture (Ac), une séquence d'acide nucléique simple brin modifiée notamment par au moins une moitié de paire d'affinité et qui s'hybride à une séquence cible ; par exemple, à une extrémité de la chaîne oligonucléotidique une ou plusieurs biotines peuvent être fixées. L'amorce de capture peut également être un oligonucléotide branché.

On entend par amorce de détection (Ad), une séquence d'acide nucléique simple brin modifiée par un système de détection et qui s'hybride à une séquence cible ; par exemple, l'oligonucléotide peut être marqué en son extrémité 5' par un phosphore 32.

Selon un autre mode de mise en oeuvre avantageux de l'invention, la quantité d'amorces utilisée au cours de l'étape (c) (deuxième série d'amplifications) est nettement inférieure à la quantité d'amorces utilisée au cours de l'étape (a).

Selon une disposition avantageuse de ce mode de mise en oeuvre, la quantité d'amorces utilisée au cours de l'étape (c) est au moins cinq fois plus faible que la quantité d'amorces utilisée au cours de l'étape (a).

Le nombre de cycles de la deuxième série d'amplifications est notamment choisi en fonction de la dilution et de la nature de l'échantillon, de façon à conférer à cette deuxième série d'amplifications, un caractère exponentiel.

Selon un autre mode de mise en oeuvre avantageux de l'invention, lorsque la paire d'amorces de l'étape (c) contient une amorce de capture, l'étape de détection (2) comprend :
(d) la mise en contact de la solution d'amplification de détection avec un support approprié qui capte les fragments d'acides nucléiques porteurs de l'amorce de capture (Ac) intégrée ; et
(e) la révélation des copies d'acide nucléique cible double brin retenues sur ledit support, par tout moyen approprié.

Cette révélation peut être réalisée soit à l'aide d'une sonde de détection (amorce de détection ou hybridation ultérieure avec une sonde marquée de manière appropriée), soit à l'aide

Dans un tel mode de mise en oeuvre, le système de capture est avantageusement une moitié de paire d'affinité. Un tel système permettra à l'oligonucléotide de se fixer sur le support possédant l'autre moitié de la paire d'affinité.

On peut citer notamment comme paires d'affinité, les paires biotine-avidine ou streptavidine, dérivé de métal lourd-groupe thio, divers homopolynucléotides comme poly dG-poly dC, poly dA-poly dT et poly dA-poly dU ainsi que divers oligonucléotides de séquences spécifiques comme dans le cas, par exemple des amorces branchées et les paires antigène-anticorps. D'autres paires de composants peuvent également être utilisées si elles présentent une affinité assez forte pour permettre la liaison spécifique des amorces de capture (Ac) incorporées dans les copies de l'acide nucléique cible au support solide ; les ligands et les conjugués peuvent être parties d'une paire d'affinité.

Le système de révélation est avantageusement choisi dans le groupe qui comprend les isotopes ($^{125}$I, $^{35}$S, $^{32}$P), les fluorophores, un système fluorescent de type lanthanide, un système enzymatique colorimétrique (phosphatase alcaline, peroxydase), un système enzymatique fluorimétrique, luminescent, bioluminescent ou électrochimique et les composés qui interagissent avec un acide nucléique double brin, notamment une matière colorante ou un agent intercalant.

Selon une disposition de ce mode de mise en oeuvre, lorsqu'une amorce de détection (Ad) est intégrée dans un brin d'acide nucléique cible, au cours de l'étape (c), la détection de l'étape (e) est réalisée à l'aide de ladite amorce de détection (Ad).

Selon une autre disposition de ce mode de mise en oeuvre, lorsqu'une amorce simple (As) est intégrée dans un brin au cours de l'étape (c), la révélation de l'étape (e) est réalisée soit par hybridation des copies d'acide nucléique cible double brin retenues sur le support avec une sonde de détection appropriée, soit par mise en contact avec un composé qui interagit avec l'acide nucléique double brin cible, notamment une matière colorante ou un agent intercalant apte à produire des modifications spectrales, puis détection desdites modifications spectrales du composé qui interagit avec l'acide nucléique double brin, par tout moyen approprié.

Selon un autre mode de mise en oeuvre du procédé conforme à l'invention, lorsque la paire d'amorces de l'étape (c) ne contient pas d'amorce de capture, l'étape de détection (2) comprend :

(d) la révélation directe en phase homogène liquide desdites séquences cibles amplifiées, par mise en contact des acides nucléiques double brin, obtenus après les étapes (a), (b) et (c) définies ci-dessus, soit avec une sonde de détection appropriée, soit avec un composé qui interagit avec un acide nucléique double brin, notamment une matière colorante ou un agent intercalant apte à produire des modifications spectrales puis détection desdites modifications spectrales du composé qui interagit avec l'acide nucléique double brin, par tout moyen approprié.

Selon une disposition avantageuse de ce mode de mise en oeuvre, ledit composé est choisi parmi les substances présentant une modification physico-chimique après liaison avec l'ADN double brin.

On peut citer à titre d'exemples non limitatifs de tels composés, le 4',6-diamidino-2-phénylindole-2 (DAPI), le bisbenzimide, l'éthidium, la 9-aminoacridine, la proflavine, la quinacrine, la chloroquine, la lucanthone, l'hycanthone, la tilorone, le mAMSA, la daunomycine, l'adriamycine, l'actinomycine D, la 9-OH N-méthyl-ellipticine, la mithoxanthrone, la bléomycine, l'échinomycine, la ditercaline et les dérivés de ces composés, l'HOECHST 33 ainsi que les nucléotides modifiés de manière appropriée.

Selon une modalité avantageuse de cette disposition, le composé propre à produire des modifications spectrales est un agent intercalant fluorescent tel que, notamment, le bromure d'éthidium ou un bis-benzimide tel que l'HOECHST 33-258.

Un tel mode de mise en oeuvre a l'avantage de ne nécessiter qu'une mesure directe de l'acide nucléique double brin marqué total présent dans la solution pour caractériser la séquence recherchée.

Un tel mode de mise en oeuvre a, en outre, l'avantage d'éliminer les réactions de compétition qui se produisent dans les procédés de l'Art antérieur nécessitant une fixation sur un support du produit d'extension double brin obtenu pour la détection de l'acide nucléique.

Selon une autre disposition avantageuse de ce mode de mise en oeuvre, les dilutions successives d'un ADN à tester, et notamment d'un ADN viral à tester, sont soumises à une première série d'amplifications dans un volume convenable contenant au moins deux séquences cibles propres à servir de matrices, les quatres désoxyribonucléosides triphosphates, une quantité appropriée de Taq DNA polymérase, dans une solution tampon appropriée à pH 8,3 contenant une faible quantité (de l'ordre de 0,01 % p/v) de gélatine, les produits de la première série d'amplifications étant ensuite soumis à une deuxième étape de dilution, puis à une deuxième série d'amplifications dans un milieu d'amplifications qui est avantageusement similaire à celui mis en oeuvre dans la première série d'amplifications et qui contient au moins deux séquences cibles propres à servir de matrices, après quoi le composé propre à produire des modifications spectrales est ajouté à la solution d'amplifications obtenue à la suite de la deuxième série d'amplifications pour révéler l'ADN viral double brin éventuellement présent dans les solutions.

Le procédé conforme à l'invention présente un certain nombre d'avantages sur les procédés de l'Art antérieur :

- une spécificité accrue ;
- une diminution du bruit de fond ;

- une sensibilité accrue, dans la mesure où l'on dilue fortement l'échantillon après l'amplification d'enrichissement ; en effet, cette forte dilution permet :

  . d'être le plus souvent en phase exponentielle de l'amplification uniquement pour les séquences cibles, c'est-à-dire lorsque la quantité Q de séquences cibles obtenues peut être reliée au facteur d'amplification x et au nombre des cycles n par la formule $Q = Q_o (1 + x)^n$ ;

  . d'avoir un rapport séquences cibles/séquences non spécifiques nettement supérieur à 1 dès le début de l'amplification de détection ;

  . de diminuer les réactions de compétition liées aux produits d'extension des acides nucléiques hétérologues et aux composants présents dans le milieu biologique (débris cellulaires, protéines, biotine naturelle , acide nucléique total initial...).

Le procédé conforme à l'invention permet également de procéder, au cours de l'amplification de détection et ce en même temps et dans le même tube à essai, à l'amplification de détection de plusieurs séquences cibles à condition d'utiliser comme amorce de capture des oligonucléotides branchés. Les duplex comportant des oligonucléotides branchés ont un brin d'ADN monocaténaire libre. Ce brin libre d'ADN simple brin permet de réaliser à l'aide d'un oligonucléotide complémentaire attaché à un support, une fixation sélective.

Il faut en outre souligner que la dilution effectuée après la première amplification permet de travailler avec une concentration en amorces assez faible pour éliminer l'incidence du phénomène de dimérisation et d'amplification des amorces qui conduirait à un signal parasite ; de plus, la dilution permet d'utiliser moins d'amorces et donc d'avoir une meilleure spécificité dans la phase de révélation de l'amplification de détection.

Le procédé conforme à l'invention est notamment applicable à la détection des maladies génétiques, des maladies infectieuses (virus, parasites, champignons, bactéries...)et des maladies tumorales tant humaines, animales que végétales, au contrôle des échantillons biologiques ainsi qu'au typage cellulaire.

La présente invention a, en outre, pour objet, un coffret, kit ou ensemble coordonné, prêt à l'emploi, pour la mise en oeuvre du procédé conforme à l'invention, caractérisé en ce qu'outre des quantités utiles de tampons et de réactifs appropriés pour la mise en oeuvre de ladite détection, il comprend au moins :

- des doses appropriées d'au moins une première paire d'amorces appropriées, pour la réalisation de l'étape (a) dudit procédé ;
- des doses appropriées d'au moins une deuxième paire d'amorces appropriées, pour la réalisation de l'étape (c) dudit procédé ; et éventuellement
- des doses appropriées d'une sonde appropriée, pour la réalisation de l'étape (e) dudit procédé et/ou
- des doses appropriées d'au moins un composé se liant de manière non covalente sur un acide nucléique double brin tel que, notamment, une matière colorante ou un agent intercalant, apte à produire des modifications spectrales lorsqu'il est fixé sur un acide nucléique ou lorsqu'il n'est pas fixé sur un acide nucléique.

Outre les dispositions qui précèdent, l'invention comprend encore d'autres dispositions, qui ressortiront de la description qui va suivre, qui se réfère à des exemples de mise en oeuvre du procédé objet de la présente invention.

Il doit être bien entendu, toutefois, que ces exemples sont donnés uniquement à titre d'illustration de l'objet de l'invention, dont ils ne constituent en aucune manière une limitation.

Dans les exemples qui suivent, les exemples 1 à 8 décrivent des modes de mises en oeuvre de l'hybrido essai conforme à l'invention et les exemples 9 à 11 décrivent des modes de mise en oeuvre en phase homogène (détection en présence d'un agent intercalant).

I - Exemples du mode de mise en oeuvre en solution.

A) détection par composé radioactif :

**Exemple 1 : Détection du virus de papillome humain de type 16 (HPV 16) à l'aide du procédé conforme à l'invention, utilisant deux amorces simples (As1et As2) pour l'amplification d'enrichissement et une paire d'amorce Ad-Ac pour l'amplification de détection.**

Lors de l'hybrido-essai conforme à l'invention, le nombre de cycles au cours de l'amplification d'enrichissement, la dilution et le nombre de cycles au cours de l'amplification de détection dépendent de la séquence cible à amplifier et à détecter de la dilution et de la nature de l'échantillon.

L'amplification d'enrichissement peut comprendre, à titre d'exemple non limitatif, entre 20 et 40 cycles, la dilution peut être comprise entre 1/200è et 1/100 000è et l'amplification de détection peut comprendre de 3 à 15 cycles ; en ce cas, les concentrations en amorce, lors de l'amplification de détection sont dix fois plus faibles que celles de l'amplification d'enrichissement, tout en obtenant dans le produit final un taux d'incorporation très élevé d'amorce (environ 75 %).

Un autre exemple de mise en oeuvre du procédé conforme à l'invention comprend deux séries d'amplification d'enrichissement qui peuvent, à titre d'exemple non limitatif, être envisagées comme suit :

- 1ère amplification d'enrichissement (10-40 cycles), dilution 1/200è ;
- 2ème amplification d'enrichissement à partir de la solution obtenue (10-40 cycles), dilution 1/1000è ;
- amplification de détection (3-15 cycles).

- Préparation du support :

Les supports utilisés ici sont des tubes en polystyrène à ailettes recouverts dans un premier temps par de la sérum-albumine-bovine (BSA) liée à de la biotine puis, dans un second temps, par de l'avidine.

5 g de BSA-biotine dans 500 µl de tampon phosphate (0,05 M pH 7,3) sont incubés 2 heures à température ambiante dans les tubes à ailettes. Le liquide est vidé et remplacé par 500 µl d'une solution d'avidine à 5 mg/ml dans le même tampon qu'on laisse incuber deux heures à température ambiante. Les sites libres du support sont ensuite bloqués par une solution à 20 µg/ml d'ADN de sperme de hareng dénaturé et sonifié. Les tubes peuvent être conservés dans ce milieu plusieurs semaines à +4°C.

- L'amplification d'enrichissement :

La solution d'ADN à tester est soumise à la première amplification dans un milieu contenant un volume total de 50 µl : 50 pmoles de chacune des deux amorces As1 et As2, 300 M de chacun des quatre désoxyribonucléosides triphosphates (c'est-à-dire dATP, dCTP, dGTP et dTTP) (Boehringer Mannheim), 1,5 mM de $MgCl_2$, 50 mM de KCl, 10 mM de Tris-HCl pH 8,9, 0,01 % (P/V) de gélatine et 2,5 unités de Taq DNA polymérase (Amersham). Trente cycles d'amplification sont effectués grâce à un appareil automatique (un cycle correspondant à 90 s de dénaturation à 92°C, 90 s d'hybridation à 50°C et 120 s de polymérisation à 72°C).

- L'amplification de détection :

2 µl de chaque échantillon préalablement amplifié dans un volume de 50 µl sont dilués dans 400 µl d'eau. 2 µl de cette solution (soit un 1/5000e de l'amplification d'enrichissement) sont ensuite soumis à l'amplification de détection de 12 cycles en un volume total de 25 µl dans le milieu d'amplification précédemment décrit en présence des amorces modifiées : soit 5 pmoles d'oligonucléotide biotinylé en 5' Ac3 et 5 pmoles d'oligonucléotide Ad4 marqué en 5' par du $^{32}P$, l'activité spécifique de ladite sonde étant volontairement limitée à 50 000 cpm/pmole ; l'activité totale par échantillon est de 250 000 cpm environ.

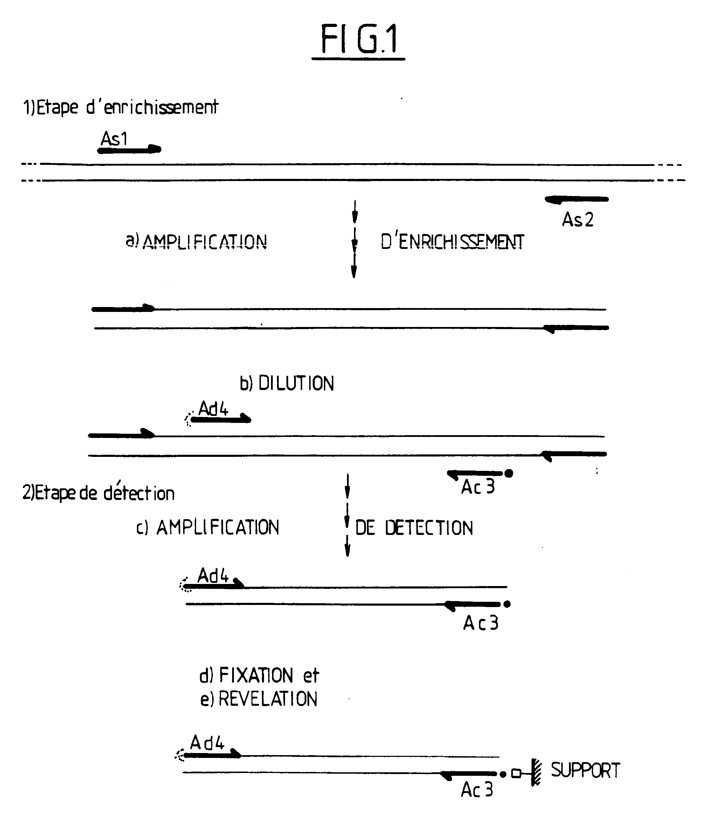

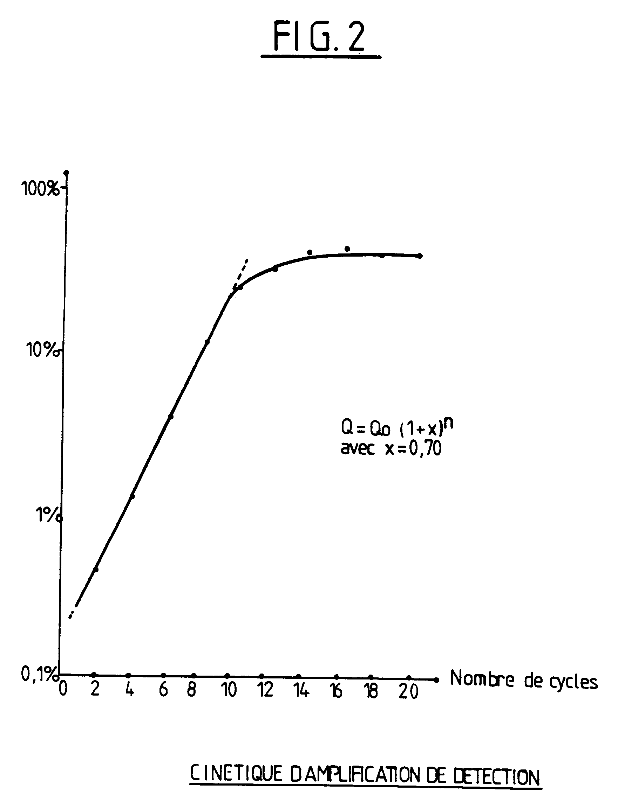

La figure 1 montre le principe du procédé décrit dans l'exemple 1. La paire d'amorces de l'amplification d'enrichissement est représentée par les amorces simples As1 et As2 ; la paire d'amorces de l'amplification de détection est représentée par les amorces Ad4 marquées en 5' au $^{32}P$ et l'amorce Ac3 biotinylée.

- Fixation sur tube et détection de l'ADN produit :

La totalité des 25 µl issue de l'amplification de détection est transférée dans un tube recouvert d'avidine contenant 475 µl de tampon de fixation ST (NaCl 0,5 M, Tris-HCl 50 mM pH 9,5). La fixation se déroule en une heure à 50°C. Les tubes sont ensuite vidés et rincés par 1 ml de ST-Tween 20 à 1 %, une fois 5 min à température ambiante, une fois 5 min à 50°C et une fois 5 min à 50°C par 1 ml de ST 0.1x-Tween 20 à 1 %.

La radioactivité fixée sur les tubes est ensuite comptée directement par effet Cerenkov. Les résultats sont exprimés par le rapport "R" de l'activité fixée dans le tube sur l'activité totale de départ (soit 250 000 cpm environ).

**Exemple 2 : Détection du virus du papillome humain de type 16 (HPV 16) à l'aide du procédé conforme à l'invention utilisant deux amorces simples As1 et As2 pour l'amplification d'enrichissement, une paire Ac3-As4 pour l'amplification de détection et une sonde Ad5 pour la détection.**

On procède comme dans l'exemple 1 ; la différence porte sur la paire d'amorces de l'amplification de détection et sur la détection.

L'oligonucléotide As4 n'est pas marqué est la révélation est assurée, après dénaturation de l'échantillon à 100°C, par une hybridation avec 0,1 pmole de sonde Ad5 (35-mer) marquée en 5' par du $^{32}P$. L'activité spécifique de la sonde est forte ($2,5.10^6$ cpm/pmole) et l'activité totale par échantillon est de 250 000 cpm environ.

La sonde marquée Ad5 de révélation est ajoutée après transfert de la solution d'amplification de détection dans un tube recouvert d'avidine contenant 475 µl de tampon de fixation ST, comme précisé dans l'exemple 1.

### Exemple 3 : Mise en évidence de l'ADN d'HPV 16 dans des biopsies suspectes du col de l'utérus.

L'ADN des biopsies est extrait classiquement au phénol-chloroforme après lyse de la cellule et digestion des protéines (Maniatis et coll, Molecular cloning, A laboratory manual, Cold Spring Harbor Laboratory, 1982). Sur les 12 échantillons, 4 étaient fortement positifs (R > 25 %), 3 étaient positifs (R > 10 %) ; les négatifs gardant un bruit de fond très bas (R < 1 %) (R étant le rapport de l'activité fixée dans le tube sur l'activité totale de départ). Les valeurs issues de cette technique conforme à l'invention sont corrélées avec les résultats obtenus par hybridation classique sur filtre, mais présentent, de manière inattendue, une sensibilité nettement supérieure.

### Exemple 4: Mise en évidence d'HPV 16 dans des écouvillonnages vaginaux.

Les écouvillonnages vaginaux sont simplement remis en suspension dans du PBS stérile puis centrifugés. Le culot est repris par 200 µl d'eau et porté à 100 °C pendant 15 min. Le surnageant est récupéré et congelé à -20 °C. Sur 8 échantillons traités selon le protocole précédemment décrit (exemple 1), un seul s'est révélé faiblement positif (R = 2.5 %), les autres ayant une activité comparable à celle des témoins négatifs (R < 1 %) ; ces résultats étant toujours corrélés avec ceux issus de la technique d'hybridation sur filtre.

### Exemple 5: Utilisation d'une amorce marquée à l'iode[125].

De l'ADN de cellules infectées par le virus HPV16 et de l'ADN de cellules non infectées, utilisé comme témoin négatif, ont été soumis à une amplification d'enrichissement selon le protocole décrit dans l'exemple 1.

Après dilution au 5000e, l'amplification de détection est effectuée en présence de l'amorce de capture Ac biotinylée et d'un oligonucléotide de détection Ad marqué à l'iode[125] en 5' selon la technique décrite dans le Brevet français 88 08240. Le marquage est limité volontairement à une activité spécifique faible (375 000 cpm-/mole). La détection du duplex se déroule selon le protocole utilisé pour une amorce marquée au $^{32}$P décrit dans l'exemple 1. Le double brin est fixé par la biotine sur le tube couvert d'avidine. Après rinçage, l'iode[125] est mesurée par un compteur gamma.

| Résultats | Rendement de comptage R | (cpm fixés/cpm total) |
|---|---|---|
| T1 (+) | (ADN de biopsie HPV16 positif) | 21,5 % |
| T2 (+) | (ADN de biopsie HPV16 positif) | 16,5 % |
| T (-) | (ADN cellulaire HPV16 négatif) | 0,4 % |
| Bruit de fond en absence d'ADN | | 0,04 % |

B) Détection non-radioactive :

## Exemple 6 : Révélation colorimétrique sur plaques de microtitration. Utilisation d'une amorce portant le groupement dinitrophényle.

De l'ADN de cellules infectées par le virus HPV16 et de l'ADN génomique sain ont été soumis à une amplification d'enrichissement, puis dilués selon le protocole décrit (voir exemple 1). L'amplification de détection est conduite en présence de l'amorce de capture Ac biotinylée et d'une amorce Ad portant en 5' le groupement dinitrophényle introduit selon la technique décrit dans le Brevet français 88 08240. La détection du duplex comporte plusieurs étapes :

1. Incubation avec un anticorps antidinitrophényle issu du lapin (Sigma Chemical Co) dans un tampon PBS (Phosphate Buffered Saline), Tween 20 1 %, BSA 1 % (Bovine Serum Albumine), 1 heure à 37°C.
2. Lavages par 3 x 400 µl de tampon PBS Tween 20 1 %, BSA 1 %, à température ambiante.
3. Incubation avec un anticorps dilué à 1/300e antilapin couplé à la phosphatase alcaline (Sigma Chemical Co), dans le même tampon, 1 heure à 37°C.
4. Trois lavages avec du PBS, Tween 20 1 %, BSA 1 %, à température ambiante.
5. Révélation par hydrolyse du phosphate de dinitrophényle (kit Sigma). Lecture à 405 nm.

| Résultats | DO | Signal/Bruit |
|---|---|---|
| T1 (+) (ADN de biopsie HPV16 positif) | 2,6 | 21,6 |
| T2 (+) (ADN de biopsie HPV16 positif) | 1,9 | 15,8 |
| T (-) (ADN de biopsie HPV16 négatif) | 0,16 | 1,3 |
| En absence d'ADN | 0,12 | 1 |

## Exemple 7 : Autres exemples de détection par le procédé en solution.

a) Amplifications :

Des extraits cellulaires bruts ont été préparés à partir de cellules Hela (HPV18 +) et de fibroblastes (HPV -) : après centrifugation, le culot cellulaire est repris par 250 µl d'eau et chauffé dix minutes à 100°C. On élimine les débris cellulaires par une courte centrifugation, et on récupère le surnageant qui contient les acides nucléiques solubles.

Une amplification d'enrichissement en HPV18 est effectuée dans un milieu PCR classique, en présence de 50 pmoles de chacune des amorces. Elle comporte 30 cycles : dénaturation 94°C 1 min 30, hybridation 50°C 1 min et élongation 72°C 1 min 30.

Une amplification de détection est conduite à partir d'une dilution au 1/800ème de la solution d'enrichissement. Cette amplification est effectuée avec deux amorces modifiées : la première comporte en 5' un résidu dinitrophényle, c'est l'amorce de capture, la deuxième un résidu biotine, c'est l'amorce de détection. Le milieu d'amplification contient 5 pmoles de chaque amorce dans un volume total de 25 µl. Le nombre de cycles est de 18 : dénaturation 94°C 1 min, hybridation 55°C 1 min et élongation 72°C 1 min.

b) Saturation des tubes :

Des tubes Nunc Startubes petit modèle sont recouverts avec des anticorps anti-DNP dans du tampon carbonate/bicarbonate de sodium 0,05 M, pH 9,5, $MgCl_2$ 1 mM, pendant une nuit à 37°C. Les sites libres sont ensuite saturés par une solution de BSA 2 % dans le même tampon pendant 3 heures à température ambiante. Les tubes sont ensuite lavés 5 minutes dans du PBS, et conservés dans ce même tampon à 4°C.

c) Fixation des produits d'amplification :

On fixe 15 µl des produits d'amplification de détection dans 500 µl de PBS pendant deux heures à 37 °C. On effectue ensuite un lavage de 5 min dans 500 µl de PBS/BSA, 0,1 %/Tween 20 4 % puis deux lavages de 5 min dans du PBS.

On procède ensuite à une saturation courte de BSA (solution à 3 % dans du PBS) : une heure à 37°C. On lave 5 min avec du PBS.

On fait réagir la solution d'avidine-phosphatase alcaline (dilution au 1/12500ème dans du PBS). La fixation de ce complexe se fait en trente minutes à température ambiante. Elle est suivie par trois lavages de 5 min dans du PBS.

d) Détection par fluorescence :

Le substrat de la phosphatase alcaline utilisé est le 4-méthylumbelliféryl phosphate. A la suite de la coupure du phosphate par l'enzyme, le substrat devient fluorescent. La longueur d'onde d'excitation est de2 374 nm et la longueur d'onde d'émission de la fluorescence est de 450 nm.

On prépare une solution stock de substrat 0,05 M dans du tampon TRIS 0,1 M, pH 9,5, NaCl 0,1 M, MgCl$_2$ 5 mM. La solution de travail est obtenue par dilution au 1/300ème de la solution stock. On en utilise 500 µl par tube. On laisse réagir l'enzyme 15 min puis on mesure la fluorescence bleue très intense.

Réglage du fluorimètre :     0 % sur le tampon
                             100 % sur l'ADN positif
Mesure de l'ADN négatif :    2 %
Mesure du blanc :            2 %

e) Détection par luminescence :

Le substrat de luminescence est un dérivé des dioxétanes : l'adamantyl-1,2-dioxétane phosphate. La luminescence est directement dépendante de l'action de l'enzyme : la coupure du phosphate provoque la formation d'un anion instable qui se scinde. Cette coupure crée un nouvel anion activé qui est responsable de la lumière émise.

On dispose d'une solution stock à 10 mg/ml. Cette solution est diluée 100 fois dans du tampon carbonate/bicarbonate de sodium 0,05 M, pH 9,5, MgCl$_2$ 1 mM. On en utilise 500 µl par tube. On laisse réagir 10 min puis on mesure la lumière dans un luminomètre.

Signal de l'ADN positif :    245
Signal de l'ADN négatif :    4
Signal du blanc :            1,8

Cette méthode de détection atteint presque les valeurs de sensibilité des méthodes radioactives. Elle est une méthode de choix pour une détection non-radioactive.

f) Détection par colorimétrie :

Le substrat utilisé pour la phosphatase alcaline est le p-nitrophényl phosphate. La perte du phosphate par action enzymatique conduit à un composé jaune dont on mesure la densité optique à 405 nm.

On dissout deux pastilles de substrat phosphatase Sigma 104 dans 10 ml de tampon diéthanolamine 0,8 M, MgCl$_2$ 5 mM, pH 9,8. On utilise 500 µl par tube. On laisse réagir 10 minutes à l'obscurité, et on arrête la réaction par addition de 250 µl de soude 2N.

Mesure de l'ADN positif :    0,32 O.D.
Mesure de l'ADN négatif :    0,02 O.D.
Mesure du blanc :            0,01 O.D.

II - Exemples du mode de mise en oeuvre en phase homogène.

**Exemple 8: Détection de l'ADN du virus de papillome humain de type 16 (HPV 16) à l'aide du mode de mise en oeuvre en phase homogène conforme à l'invention.**

Les différentes solutions d'ADN à tester ont été soumises à une première amplification dans un milieu contenant en un volume total de 50 µl : 50 pmoles de chacune des deux amorces A1 et A2, 200 µM de chacun des quatre désoxyribonucléosides triphosphates (c'est-à-dire dATP, dCTP, dGTP et dTTP) ( Boehringer Mannheim), 1,5 mM de MgCl$_2$, 50 mM de KCl, 10 mM de Tris-HCl pH 8,3, 0,01 % (P/V) de gélatine et 2,5 unités de Taq DNA polymérase (Amersham). Trente cycles d'amplification sont effectués grâce à un appareil automatique (un cycle correspondant à 90 s de dénaturation à 94°C, 90 s d'hybridation à 50°C et 120 s de polymérisation à 72°C).

1 µl de chaque échantillon préalablement amplifié est dilué dans 100 µl d'eau. 1 µl de chaque solution (1/2 500e) est ensuite soumis à une deuxième série d'amplifications de 15 cycles en un volume total de 25 µl dans le milieu d'amplification précédemment décrit et contenant 5 pmoles des deux amorces B1 et B2.

A 20 µl de solution d'amplification, on ajoute 1 µl de bromure d'éthidium à 5 µg/ml (soit 5 ng) ; on agite, puis on observe les tubes sous U.V., à 254 nm. Seules les solutions contenant de l'ADN viral de HPV16 émettent une fluorescence observable. Cela permet une discrimination rapide entre les échantillons positifs et négatifs.

Les résultats obtenus correspondent à ceux obtenus avec le gel de contrôle.

**Exemple 9: Détection d'ADN viral de HPV18 contenu dans un plasmide.**

On prépare des dilutions successives d'ADN plasmidique de pBr322 contenant ou non l'ADN viral de HPV18. On obtient ainsi les concentrations de 20, 10, 1 et 0,1 ng d'ADN pour 1 µl.

Les huit solutions d'ADN ainsi obtenues sont soumises à amplification dans un volume de 25 µl contenant 3 pmoles de chacune des deux amorces $A_1$ et $A_2$, les quatre desoxyribonucléosides triphosphate (200 µM), du $MgCl_2$ 1,5 mM, du KCl 50 mM, du TRIS-HCl pH 8,3 10 mM, de la gélatine 0,01 % (p/v) et 0,25 unité de Taq DNA polymérase. Quinze cycles d'amplification sont effectués à l'aide d'un appareil automatique : dénaturation à 94°C pendant 1 minute 30, hybridation à 55°C pendant 1 minute 30 puis élongation à 72°C pendant 1 minute.

A la fin de ces quinze cycles, on ajoute dans chacun des tubes 1 µl de solution de bromure d'éthidium à 5 µg/ml (soit 5 ng), on agite rapidement puis on observe les tubes sur une table lumineuse UV 254 nm.

Les tubes contenant les solutions d'amplification de plasmide avec l'ADN viral (20, 10 et 1 ng) fluorescent nettement, celui contenant 0,1 ng de plasmide avec ADN viral fluoresce légèrement et ceux contenant le plasmide seul ne donnent aucun signal.

La détection au bromure d'éthidium du produit de l'amplification d'un ADN très purifié et très dilué est réalisée immédiatement après l'amplification et donne un résultat très significatif.

**Exemple 10: Autres exemples de détection en milieu liquide direct :**

1) Utilisation d'un bis-benzimide (dénommé "HOECHST 33-258") :
a) détection de l'ADN du virus du HIV (Syndrome de l'Immuno Déficience Acquise).

1 µl d'ADN provenant de lymphocytes ou monocytes de sujets sains ou porteurs du virus, de rate de sujet porteur du virus, de plasmide contenant un insert provenant de la souche HIVI/lav sont soumis à une amplification primaire, dans un volume de 50 µl, comme décrit dans l'exemple 1.

1 µl de chaque échantillon préalablement amplifié est dilué dans 200 µl d'$H_2O$. 2 µl de cette solution sont soumis à une deuxième amplification de 25 cycles dans un volume final de 25 µl avec les amorces B1 et B2.

20 µl de cette solution sont ensuite dilués dans 1 ml de tampon Tris HCl 50 mM, pH 7,4 NaCl 2M contenant 1 µl final de HOECHST 33-258.

Après agitation, la fluorescence est mesurée dans un spectrifluorimètre Perkin Elmer LS5 (excitation 360 nm, émission 450 nm). L'appareil est préalablement étalonné avec différentes dilutions d'ADN double brin.
gamme étalon :

```
0,75 µg     0,35 µg     0,19 µg     0,01 µg     0 µg     ADN
100 %       30 %        15 %        9 %         0 %
```

On utilise deux jeux de primers différents dans le gène tat et dans le gène pol de HIV1.

Résultats de pol :

|  | TEMOIN $H_2O$ | LYMPHO+ | LYMPHO- | RATE+ | PLASMIDE + |
|---|---|---|---|---|---|
| SOLUTION | – | ++ | – | ++ | +++ |
| FILTRE | – | ++ | – | ++ | +++ |

Résultats de tat :

|  | TEMOIN H$_2$O | LYMPHO+ | LYMPHO- | MONO+ | PLASMIDE + |
|---|---|---|---|---|---|
| SOLUTION | - | ++ | - | + | +++ |
| FILTRE | - | ++ | - | - | +++ |

b) Détection de l'ADN du virus du Papillome Humain de types 16 et 18.

5 µl d'une solution de surnageant de cellules HPV 18+ (HELA) et HPV 16+ (CASKI), ($10^6$ cellules dans 250 µl d'H$_2$0), et de fibroblastes négatifs pour HPV, sont soumis à une amplification comme décrit précédemment. Après une dilution au 200ème, 2 µl sont soumis à une amplification secondaire et mesuré par fluorescence.

gamme étalon :

| 1,5 µg | 0,75 µg | 0,38 µg | 0,19 µg | 0,1 µg | 0 µg ADN |
|---|---|---|---|---|---|
| 100 % | 48 % | 20 % | 8 % | 2 % | 0 % |

HPV 16 :

|  | TEMOIN | FIBROBLASTES | CASKI |
|---|---|---|---|
| SOUTHERN | - | - | +++ |
| SOLUTION | - | - | +++ |

HPV 18 :

|  | TEMOIN | FIBROBLASTES | HELA |
|---|---|---|---|
| SOUTHERN | - | - | +++ |
| SOLUTION | - | - | +++ |

2) Utilisation du DAPI :

a) Détection de HPV 18 :

Même protocole que celui de l'exemple précédent ; on teste deux dilutions différentes : 1/50 et 1/200.

La fluorescence est mesurée dans un tampon Tris HCl 5 mM, pH 7,6, NaCl 8 mM, contenant 0,2 µg/ml de DAPI (excitation 372 nm, émission 454 nm).

gamme étalon :

| 1,5 µg | 0,75 µg | 0,38 µg | 0,19 µg | 0,1 µg | 0 µg |
|---|---|---|---|---|---|
| 100 % | 50 % | 24 % | 12 % | 6 % | 0 % |

12

HPV 18 :

|  | TEMOIN | FIBROBLASTES |
|---|---|---|
| SOLUTION 1/200 | ++ | - |
| SOUTHERN | ++ | - |

b) Détection de HIV1 :
Même protocole que exemples précédents.
gamme étalon :

|  | 2 μg | 1 μg | 0,5 μg | 0,25 μg | 0,12 μg | 0 μg |
|---|---|---|---|---|---|---|
| % | 100 | 48 | 20 | 10 | 6 | 0 |

HIV1 :

|  | TEMOIN | LYMPHO+ | MONO+ | LYMPHO- | PLASMIDE+ |
|---|---|---|---|---|---|
| SOUTHERN | - | ++ | - | - | +++ |
| SOLUTION | - | ++ | + | - | +++ |

III - Rôle de l'étape (b) de dilution.

Des expériences préliminaires ont montré qu'une dilution au 1/10è entraînent un bruit de fond important avec un signal bas après la liaison des hybrides à la matrice d'affinité et ce, indépendamment du nombre de cycles de la deuxième série d'amplifications. Ceci peut s'expliquer par le fait que la PCR est habituellement réalisée en présence d'un excès important d'amorces et qu'après une dilution au 1/10è, la quantité d'amorces d'amplification encore présente en solution, est élevée. Au cours de la deuxième série d'amplifications, il se produit alors une compétition entre les amorces d'amplification et les amorces de détection conduisant à une diminution de l'efficacité de la PCR. Des concentrations élevées en amorces peuvent également conduire à la formation d'amorces dimères.

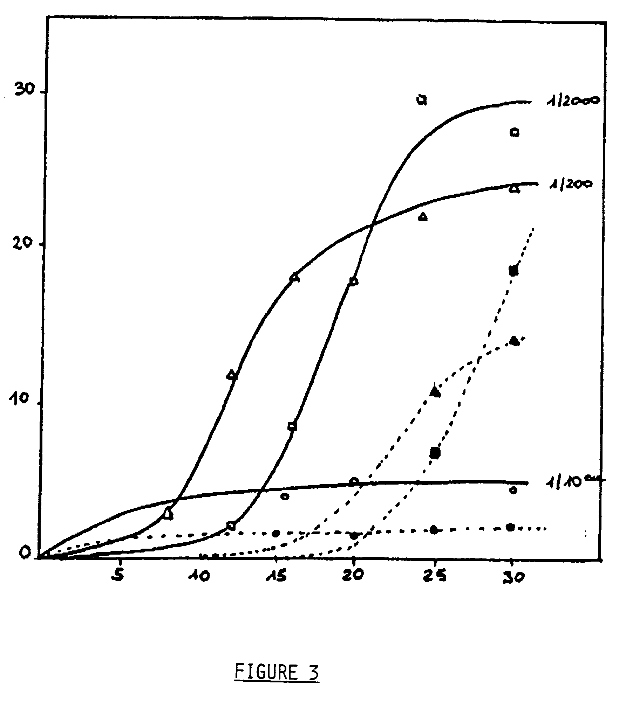

La présence de débris cellulaires peut également conduire à une inhibition partielle de la réaction lorsque la PCR est réalisée sur des échantillons d'ADN brut et une dilution au 1/10è n'est pas suffisante pour les éliminer. Les valeurs de bruit de fond sont obtenues en mesurant l'amplification de séquences non-spécifiques. Le signal spécifique est alors défini comme le nombre de coups d'une amplification spécifique après soustraction de la valeur de bruit de fond. On estime la sensibilité de la procédure par la mesure du pourcentage de fixation de signal spécifique. Les résultats sont représentés sur la figure 3. La sensibilité de détection est établie en fonction du nombre de cycles.

La figure 3 qui comporte en abscisse le nombre de cycles et en ordonnée le pourcentage de fixation montre des PCR réalisées sur des cellules HPV 18+ (Hela Δ, □, ○) et des cellules HPV 18- (BJAB, ■, ▲, ●). La première amplification est réalisée sur des cellules (2.10⁴) dans un volume de 100 μl. Après une dilution au 1/10e (○, ●), une dilution au 1/200è (Δ, ▲) et au 1/2 000e (□, ■), les échantillons sont soumis à une deuxième amplification en présence d'une amorce marquée à l'iode 125 et d'une amorce biotinylée. Les hybrides sont récupérés et la radioactivité est mesurée. L'activité totale est de 71 000 cpm/tube, la valeur représentée est le pourcentage de radioactivité fixée.

Lorsque la dilution est portée respectivement au 1/200e (Δ, ▲), et au 1/2 000e (□, ■), le nombre de copies de la séquence cible présentes au moment de la deuxième amplification sont initialement inférieures et entraînent une meilleure efficacité de la PCR. La phase exponentielle est alors plus importante et le plateau est atteint après 20 et 25 cycles respectivement. En comparaison le bruit de fond commence à augmenter exponentiellement après 16 et 20 cycles.

## Revendications

1. Procédé de détection de séquences spécifiques d'acide(s) nucléique(s) (séquence unique et/ou mélange de séquences d'acides nucléiques) présente(s) dans un échantillon biologique, comprenant au moins une amplification enzymatique, caractérisé en ce qu'après mise en solution appropriée de l'échantillon biologique pour extraire le/les acides nucléiques, ledit procédé comprend la détection d'une séquence d'acide nucléique ou d'un mélange de séquences à l'aide des étapes suivantes :

   (1) une étape d'enrichissement en séquence(s) cible(s) par :

   (a) mise en contact de l'échantillon biologique mis en solution, avec au moins une paire d'amorces appropriées, pour amplifier au moins un fragment de ladite/desdites séquences d'acide nucléique cible, lesdites amorces s'hybridant à ladite/lesdites séquences cibles et permettant d'obtenir une solution d'amplification d'enrichissement ; et

   (b) au moins une dilution, comprise entre 1/50e et 1/100 000e, de la solution d'amplification d'enrichissement obtenue en (a) ;

   (2) une étape de détection des séquences cibles amplifiées obtenues par :

   (c) mise en contact d'une fraction de la solution d'enrichissement obtenue en (b) avec au moins une paire d'amorces dont au moins l'une des séquences est incluse dans la séquence cible amplifiée en (a) pour amplifier au moins un fragment de ladite/desdites séquences d'acide nucléique cible ; et

   (d) détection des copies d'acide nucléique cible double brin obtenues en (c), par tout moyen approprié.

2. Procédé selon la revendication 1, caractérisé en ce que les étapes (a) et (b) sont répétées au moins une fois.

3. Procédé selon la revendication 1 ou la revendication 2, caractérisé en ce que la dilution de l'étape (b) est comprise entre 1/50e et 1/50 000e.

4. Procédé selon la revendication 3, caractérisé en ce que la dilution de l'étape (b) est comprise entre 1/50e et 1/10 000e, de préférence entre 1/200e et 1/5 000e.

5. Procédé selon l'une quelconque des revendications 1 à 4, caractérisé en ce que les amorces de l'étape (a) sont des séquences nucléotidiques simple brin (amorce simple (As)), qui s'hybrident à la séquence cible appropriée.

6. Procédé selon l'une quelconque des revendications 1 à 5, caractérisé en ce que les amorces de l'étape (c) sont des paires choisies dans le groupe qui comprend les paires : amorce modifiée par un système de capture-amorce modifiée par un système de détection (Ac-Ad), les paires amorce simple-amorce simple (As1-As2), les paires amorce simple-amorce modifié par un système de capture (As1-Ac), les paires amorce simple-amorce modifiée par un système de détection (As1-Ad).

7. Procédé selon l'une quelconque des revendications 1 à 6, caractérisé en ce que la quantité d'amorces utilisée au cours de l'étape (c) (deuxième série d'amplifications) est nettement inférieure à la quantité d'amorces utilisée au cours de l'étape (a).

8. Procédé selon la revendication 7, caractérisé en ce que la quantité d'amorces utilisée au cours de l'étape (c) est au moins cinq fois plus faible que la quantité d'amorces utilisée au cours de l'étape (a).

9. Procédé selon l'une quelconque des revendications 1 à 8, caractérisé en ce que lorsque la paire d'amorces de l'étape (c) contient une amorce de capture, l'étape de détection (2) comprend :

   (d) la mise en contact de la solution d'amplification de détection avec un support approprié qui capte les fragments d'acides nucléiques porteurs de l'amorce de capture (Ac) intégrée ; et

   (e) la révélation des copies d'acide nucléique cible double brin retenues sur ledite support, par tout moyen approprié.

10. Procédé selon la revendication 9, caractérisé en ce que lorsqu'une amorce de détection (Ad) est intégrée dans un brin d'acide nucléique cible, au cours de l'étape (c), la détection de l'étape (e) est réalisée à l'aide de ladite amorce de détection (Ad).

11. Procédé selon la revendication 9, caractérisé en ce que lorsqu'une amorce simple (As) est intégrée dans un brin au cours de l'étape (c), la révélation de l'étape (e) est réalisée soit par hybridation des copies d'acide nucléique cible double brin retenues sur le support avec une sonde de détection appropriée, soit par mise en contact avec un composé qui interagit avec l'acide nucléique double brin cible, notamment une matière colorante ou un agent intercalant apte à produire des modifications spectrales, puis détection desdites modifications spectrales du composé qui interagit avec l'acide nucléique double brin, par tout moyen approprié.

12. Procédé selon l'une quelconque des revendications 1 à 8, caractérisé en ce que lorsque la paire d'amorces de l'étape (c) ne contient par d'amorce de capture, l'étape de détection (2) comprend :
(d) la révélation directe en phase homogène liquide desdites séquences cibles amplifiées, par mise en contact des acides nucléiques double brin, obtenus après les étapes (a), (b) et (c) définies ci-dessus, soit avec une sonde de détection appropriée, soit avec un composé qui interagit avec un acide nucléique double brin, notamment une matière colorante ou un agent intercalant apte à produire des modifications spectrales puis détection desdites modifications spectrales du composé qui interagit avec l'acide nucléique double brin, par tout moyen approprié.

13. Procédé selon la revendication 12, caractérisé en ce que ledit composé est choisi parmi les substances présentant une modification physico-chimique après liaison avec l'ADN double brin.

14. Procédé selon la revendication 12 ou la revendication 13, caractérisé en ce que le composé propre à produire des modifications spectrales est un agent intercalant fluorescent tel que, notamment, le bromure d'ethidium ou un bis-benzimide.

15. Procédé selon l'une quelconque des revendications 12 à 14, caractérisé en ce que les dilutions successives d'un ADN à tester, et notamment d'un ADN viral à tester, sont soumises à une première série d'amplifications dans un volume convenable contenant au moins deux séquences cibles propres à servir de matrices, les quatres désoxyribonucléosides triphosphates, une quantité appropriée de Taq DNA polymérase, dans une solution tampon appropriée à pH 8,3 contenant une faible quantité (de l'ordre de 0,01 % p/v) de gélatine, les produits de la première série d'amplifications étant ensuite soumis à une deuxième étape de dilution, puis à une deuxième série d'amplifications dans un milieu d'amplifications qui est avantageusement similaire à celui mis en oeuvre dans la première série d'amplifications et qui contient au moins deux séquences cibles propres à servir de matrices, après quoi le composé propre à produire des modifications spectrales est ajouté à la solution d'amplifications obtenue à la suite de la deuxième série d'amplifications pour révéler l'ADN viral double brin éventuellement présent dans les solutions.

16. Coffret, kit ou ensemble coordonné, prêt à l'emploi, pour la mise en oeuvre du procédé selon l'une quelconque des revendications 1 à 15, caractérisé en ce qu'outre des quantités utiles de tampons et de réactifs appropriés pour la mise en oeuvre de ladite détection, il comprend au moins :
- des doses appropriées d'au moins une première paire d'amorces appropriées, pour la réalisation de l'étape (a) dudit procédé ;
- des doses appropriées d'au moins une deuxième paire d'amorces appropriées, pour la réalisation de l'étape (c) dudit procédé ; et éventuellement
- des doses appropriées d'une sonde appropriée, pour la réalisation de l'étape (e) dudit procédé et/ou
- des doses appropriées d'au moins un composé se liant de manière non covalente sur un acide nucléique double brin tel que, notamment, une matière colorante ou un agent intercalant, apte à produire des modifications spectrales lorsqu'il est fixé sur un acide nucléique ou lorsqu'il n'est pas fixé sur un acide nucléique.

17. Application du procédé selon l'une quelconque des revendications 1 à 15, à la détection des maladies génétiques tant humaines, animales que végétales.

18. Application du procédé selon l'une quelconque des revendications 1 à 15, à la détection des maladies infectieuses tant humaines, animales que végétales.

**19.** Application du procédé selon l'une quelconque des revendications 1 à 15, à la détection des maladies tumorales tant humaines, animales que végétales.

**20.** Application du procédé selon l'une quelconque des revendications 1 à 15, au contrôle des échantillons biologiques.

**21.** Application du procédé selon l'une quelconque des revendications 1 à 15, au typage cellulaire.

## Claims

**1.** Method for detecting specific nucleic acid sequences (single sequence and/or mixture of nucleic acid sequences) present in a biological sample, comprising at leat one enzymatic amplification, characterised in that, after the biological sample has been suitably dissolved to extract the nucleic acid or acids, the said method comprises the detection of a nucleic acid sequence or mixture of sequences by means of the following steps:

(1) a step of enrichment in the target sequence(s) by:

(a) bringing the biological sample which has been dissolved into contact with at least one pair of suitable primers in order to amplify at least one fragment of the said target nucleic acid sequence or sequences, the said primers hybridising with the said target sequence or sequences and enabling an enrichment amplification solution to be obtained; and

(b) at least one dilution, to between 1/50 and 1/100,000, of the enrichment amplification solution obtained in (a);

(2) a step of detection of the amplified target sequences obtained, by:

(c) bringing a fraction of the enrichment solution obtained in (b) into contact with at least one pair of primers, at least one of the sequences of which is included in the target sequence amplified in (a), in order to amplify at least one fragment of the said target nucleic acid sequence or sequences; and

(d) detection of the copies of double-stranded target nucleic acid obtained in (c), by any suitable means.

**2.** Method according to Claim 1, characterised in that steps (a) and (b) are repeated at least once.

**3.** Method according to Claim 1 or Claim 2, characterised in that the dilution of step (b) is between 1/50 and 1/50,000.

**4.** Method according to Claim 3, characterised in that the dilution of step (b) is between 1/50 and 1/10,000, and preferably between 1/200 and 1/5000.

**5.** Method according to any one of Claims 1 to 4, characterised in that the primers of step (a) are single-stranded nucleotide sequences (single primer (sP)) which hybridise with the appropriate target sequence.

**6.** Method according to any one of Claims 1 to 5, characterised in that the primers of step (c) are pairs chosen from the group comprising the following pairs: primer modified by a capture system-primer modified by a detection system (cP-dP), single primer-single primer pairs (sP1-sP2), pairs comprising single primer-primer modified by a capture system (sP1-cP), pairs comprising single primer-primer modified by a detection system (sP1-dP).

**7.** Method according to any one of Claims 1 to 6, characterised in that the quantity of primers used during step (c) (second series of amplifications) is markedly less than the quantity of primers used during step (a).

**8.** Method according to Claim 7, characterised in that the quantity of primers used during step (c) is at least five-fold less than the quantity of primers used during step (a).

**9.** Method according to any one of Claims 1 to 8, characterised in that, when the pair of primers of step (c) contains a capture primer, the detection step (2) comprises:

(d) bringing the detection amplification solution into contact with a suitable support which captures the nucleic acid fragments carrying the integrated capture primer (cP); and

(e) visualisation of the copies of double-stranded target nucleic acid retained on the said support, by any suitable means.

10. Method according to Claim 9, characterised in that, when a detection primer (dP) is integrated in a target nucleic acid strand during step (c), the detection of step (e) is carried out by means of the said detection primer (dP).

11. Method according to Claim 9, characterised in that, when a single primer (sP) is integrated in a strand during step (c), the visualisation of step (e) is carried out either by hybridisation of the copies of double-stranded target nucleic acid retained on the support with a suitable detection probe, or by bringing into contact with a compound which interacts with the target double-stranded nucleic acid, in particular a staining material or an intercalating agent capable of producing spectral modifications, followed by detection of the said spectral modifications of the compound which interacts with the double-stranded nucleic acid, by any suitable means.

12. Method according to any one of Claims 1 to 8, characterised in that, when the pair of primers of step (c) does not contain a capture primer, the detection step (2) comprises:
(d) direct visualisation of the said amplified target sequences in a homogeneous liquid phase, by bringing the double-stranded nucleic acids obtained after steps (a), (b) and (c) defined above into contact either with a suitable detection probe, or with a compound which interacts with a double-stranded nucleic acid, in particular a staining material or an intercalating agent capable of producing spectral modifications, followed by detection of the said spectral modifications of the compound which interacts with the double-stranded nucleic acid, by any suitable means.

13. Method according to Claim 12, characterised in that the said compound is chosen from substances which exhibit a physicochemical modification after binding to double-stranded DNA.

14. Method according to Claim 12 or Claim 13, characterised in that the compound capable of producing spectral modifications is a fluorescent intercalating agent such as, in particular, ethidium bromide or a bisbenzimide.

15. Method according to any one of Claims 12 to 14, characterised in that the successive dilutions of a DNA to be tested, and in particular of a viral DNA to be tested, are subjected to a first series of amplifications in an appropriate volume containing at leat two target sequences capable of serving as templates, the four deoxyribonucleoside triphosphates and a suitable quantity of Taq DNA polymerase, in a suitable buffer solution at pH 8.3 containing a small quantity (of the order of 0.01% w/v) of gelatin, the products of the first series of amplifications then being subjected to a second step of dilution, and thereafter to a second series of amplifications in an amplification medium which is advantageously similar to that employed in the first series of amplifications and which contains at least two target sequences capable of serving as templates, after which the compound capable of producing spectral modifications is added to the amplification solution obtained following the second series of amplifications in order to visualise the double-stranded viral DNA possibly present in the solutions.

16. Ready-to-use outfit, kit or coordinated combination for carrying out the method according to any one of Claims 1 to 15, characterised in that, apart from appropriate quantities of buffers and of suitable reactants for carrying out the said detection, it comprises at least:
- suitable doses of at least one first pair of suitable primers for carrying out step (a) of the said method;
- suitable doses of at least one second pair of suitable primers for carrying out step (c) of the said method; and, where appropriate,
- suitable doses of a suitable probe for carrying out step (e) of the said method, and/or
- suitable doses of at lest one compound which binds non-covalently to a double-stranded nucleic acid, such as, in particular, a staining material or an intercalating agent capable of producing spectral modifications when it is bound to a nucleic acid, or when it is not bound to a nucleic acid.

17. Application of the method according to any one of Claims 1 to 15 to the detection of genetic disorders both of humans and animals and of plants.

18. Application of the method according to any one of Claims 1 to 15 to the detection of infectious diseases both of humans and animals and of plants.

19. Application of the method according to any one of Claims 1 to 15 to the detection of neoplastic diseases both of humans and animals and of plants.

20. Application of the method according to any one of Claims 1 to 15 to the monitoring of biological samples.

21. Application of the method according to any one of Claims 1 to 15 to cell typing.

**Patentansprüche**

1. Verfahren zum Nachweis von spezifischen Sequenzen aus einer bzw. mehreren Nucleinsäure(n) (Einzelsequenz und/oder Gemisch aus Nucleinsäuresequenzen), die in einer biologischen Probe vorhanden ist bzw. sind, umfassend mindestens eine enzymatische Amplification, dadurch **gekennzeichnet, daß** man, nachdem man die biologische Probe zu Extraktion der Nucleinsäure(n) in eine geeignete Lösung gebracht hat, eine Nucleinsäuresequenz oder ein Gemisch aus Sequenzen mit Hilfe der folgenden Stufen nachweist:
   (1) eine Stufe der Anreicherung der Zielsequenz bzw. Zielsequenzen durch:
      (a) Inkontaktbringen der in Lösung gebrachten biologischen Probe mit mindestens einem Paar geeigneter Startsequenzen, um mindestens ein Fragment der Nucleinsäurezielsequenz bzw. -sequenzen zu amplifizieren, wobei die Startsequenzen mit der bzw. den Zielsequenz(en) hybridisieren und den Erhalt einer Amplificationslösung zur Anreicherung gestatten, und
      (b) mindestens eine Verdünnung zwischen 1/50 und 1/100.000 der in (a) erhaltenen Amplificationslösung zur Anreicherung;
   (2) eine Stufe des Nachweises der erhaltenen amplifizierten Zielsequenzen durch:
      (c) Inkontaktbringen einer Fraktion der in (b) erhaltenen Anreicherungslösung mit mindestens einem Paar Startsequenzen, von denen mindestens eine der Sequenzen in der amplifizierten Zielsequenz gemäß (a) enthalten ist, um mindestens ein Fragment der Nucleinsäurezielsequenz bzw. -sequenzen zu amplifizieren, und
      (d) Nachweis der doppelsträngigen, in (c) erhaltenen Kopien der Nucleinsäuren durch irgendein geeignetes Mittel.

2. Verfahren nach Anspruch 1, dadurch **gekennzeichnet**, daß die Stufen (a) und (b) mindestens einmal wiederholt werden.

3. Verfahren nach Anspruch 1 oder 2, dadurch **gekennzeichnet**, daß die Verdünnung der Stufe (b) zwischen 1/50 und 1/50.000 liegt.

4. Verfahren nach Anspruch 3, dadurch **gekennzeichnet,** daß die Verdünnung der Stufe (b) zwischen 1/50 und 1/10.000, bevorzugt zwischen 1/200 und 1/5.000, liegt.

5. Verfahren nach einem der Ansprüche 1 bis 4, dadurch **gekennzeichnet,** daß die Startsequenzen aus Stufe (a) einzelsträngige Nucleotidsequenzen (einfache Startsequenzen (As)) sind, die mit der geeigneten Zielsequenz hybridisieren.

6. Verfahren nach einem der Ansprüche 1 bis 5, dadurch **gekennzeichnet**, daß die Startsequenzen aus Stufe (c) Paare, ausgewählt aus der Gruppe, umfassend die Paare: durch ein Einfangsystem modifizierte Startsequenz durch ein Detektionssystem modifizierte Startsequenz (Ac-Ad), die Paare: einzelsträngige Startsequenz - einzelsträngige Startsequenz (As1-As2), die Paare: einzelsträngige Startsequenz - durch ein Einfangsystem modifizierte Startsequenz (As1-Ac), die Paare: einzelsträngige Startsequenz - durch ein Detektionssystem modifizierte Startsequenz (As1-Ad), sind.

7. Verfahren nach einem der Ansprüche 1 bis 6, dadurch **gekennzeichnet,** daß die Menge der im Verlauf der Stufe (c) verwendeten Startsequenzen (zweiter Amplificationscyclus) deutlich unter der Menge der im Verlauf der Stufe (a) verwendeten Startsequenzen liegt.

8. Verfahren nach Anspruch 7, dadurch **gekennzeichnet**, daß die Menge der im Verlauf von Stufe (c) verwendeten Startsequenzen mindestens fünfmal geringer als die Menge der in Stufe (a) verwendeten Startsequenzen ist.

9.  Verfahren nach einem der Ansprüche 1 bis 8, dadurch **gekennzeichnet,** daß, wenn das Paar der Startsequenzen aus Stufe (c) eine Einfangstartsequenz enthält, die Stufe des Nachweises (2) umfaßt:

    (d) Kontaktieren der Amplificationslösung zur Detektion mit einem geeigneten Träger, der die Nucleinsäurefragmente, die die integrierte Startsequenz zum Einfang (Ac) tragen, einfängt, und

    (e) Nachweis von Kopien von doppelsträngigen Zielnucleinsäuren, die auf dem Träger zurückgehalten wurden, durch jedes beliebige geeignete Mittel.

10. Verfahren nach Anspruch 9, dadurch **gekennzeichnet,** daß, wenn ein Startstück zum Nachweis (Ad) in einen Nucleinsäurezielstrang im Verlauf der Stufe (c) integriert wird, der Nachweis der Stufe (e) mit Hilfe des Startstücks zum Nachweis (Ad) durchgeführt wird.

11. Verfahren nach Anspruch 9, dadurch **gekennzeichnet,** daß, wenn eine einzelsträngige Startsequenz (As) in einen Strang im Verlauf der Stufe (c) integriert wird, der Nachweis der Stufe (e) entweder durch Hybridisieren der doppelsträngigen Kopien der Zielnucleinsäuren, die auf dem Träger zurückgehalten wurden, mit einer geeigneten Nachweissonde durchgeführt wird, oder durch Inkontaktbringen mit einer Verbindung, die mit der doppelsträngigen Zielnucleinsäure in Wechselwirkung tritt, insbesondere einem Farbstoff oder einem intercalierenden Mittel mit der Fähigkeit zur Erzeugung von Spektralmodifikationen, anschließenden Nachweis der Spektralmodifikationen der Verbindung, die mit der doppelsträngigen Nucleinsäure wechselwirkt, durch jedes geeignete Mittel, durchgeführt wird.

12. Verfahren nach einem der Ansprüche 1 bis 8, dadurch **gekennzeichnet,** daß, wenn das Paar der Startsequenzen der Stufe (c) keine Startsequenz zum Einfang enthält, die Stufe der Detektion (2) umfaßt:

    (d) Direkter Nachweis in homogener flüssiger Phase der amplifizierten Zielsequenzen durch Inkontaktbringen der doppelsträngigen Nucleinsäuren, die nach den vorstehenden Stufen (a), (b) und (c) erhalten wurden, entweder mit einer geeigneten Nachweissonde oder mit einer Verbindung, die mit einer doppelsträngigen Nucleinsäure in Wechselwirkung tritt, insbesondere einem Farbstoff oder einem intercalierenden Mittel mit der Fähigkeit zur Erzeugung von Spektralmodifikationen, anschließender Nachweis der Spektralmodifikationen der Verbindung, die mit der doppelsträngigen Nucleinsäure in Wechselwirkung trat, durch jedes beliebige geeignete Mittel.

13. Verfahren nach Anspruch 12, dadurch **gekennzeichnet,** daß die Verbindung aus Substanzen, die nach der Bindung an doppelsträngige DNA eine physikalischchemische Modifikation zeigen, ausgewählt wird.

14. Verfahren nach Anspruch 12 oder 13, dadurch **gekennzeichnet,** daß die Verbindung mit der Eignung zur Erzeugung von Spektralmodifikationen ein fluoreszierendes, intercalierendes Mittel, wie insbesondere Ethidiumbromid oder Bis-Benzimidin, ist.

15. Verfahren nach einem der Ansprüche 12 bis 14, dadurch **gekennzeichnet,** daß die schrittweisen Verdünnungen einer zu testenden DNA, und insbesondere einer zu testenden viralen DNA, einem ersten Amplificationscyclus in einem geeigneten Volumen, das mindestens zwei Zielsequenzen mit der Eignung, als Matrizen zu dienen, die vier Desoxyribonucleosidtriphosphate, eine geeignete Menge an Taq DNA-Polymerase in einer geeigneten Pufferlösung mit pH 8,3, die eine geringe Menge (in der Größenordnung von 0,01% Gew./Vol.) Gelatine enthält, enthält, unterworfen werden, wobei die Produkte des ersten Amplificationscyclus anschließend einem zweiten Verdünnungsschritt, dann einem zweiten Amplificationscyclus in einem Amplificationsmedium, das bevorzugt dem in dem ersten Amplificationscyclus verwendeten ähnlich ist, und das mindestens zwei Zielsequenzen mit der Eignung, als Matrizen zu dienen, enthält, unterworfen werden, wonach die Verbindung mit der Eignung zur Erzeugung von Spektralmodifikationen der nach dem zweiten Amplificationscyclus erhaltenen Amplificationslösung zugesetzt wird, um virale doppelsträngige DNA, die in den Lösungen gegebenenfalls vorhanden ist, nachzuweisen.

16. Gebrauchsfertige Reagenszusammenstellung, Kit oder aufeinander abgestimmte Garnitur zur Durchführung des Verfahrens nach einem der Ansprüche 1 bis 15, dadurch **gekennzeichnet,** daß sie bzw. es außer nützlichen Mengen an Puffern und Reagenzien zur Durchführung eines Nachweises mindestens:

    - geeignete Mengen mindestens eines ersten Paares geeigneter Startsequenzen zur Durchführung der Stufe (a) des Verfahrens;

    - geeignete Mengen mindestens eines zweiten Paares geeigneter Startsequenzen zum Nachweis der Stufe (c) des Verfahrens, und gegebenenfalls

    - geeignete Mengen einer geeigneten Sonde zur Durchführung der Stufe (e) des Verfahrens und/oder

- geeignete Mengen mindestens einer Verbindung, die sich nicht covalent an eine doppelsträngige Nucleinsäure bindet, wie insbesondere eines Farbstoffs oder eines intercalierenden Mittels, mit der Eignung zur Erzeugung von Spektralmodificationen bei Fixierung an eine Nucleinsäure oder bei fehlender Fixierung an eine Nucleinsäure,

umfaßt.

17. Verwendung des Verfahrens nach einem der Ansprüche 1 bis 15 zum Nachweis genetischer Erkrankungen sowohl von Menschen und Tieren als auch von Pflanzen.

18. Verwendung des Verfahrens nach einem der Ansprüche 1 bis 15 zum Nachweis von Infektionskrankheiten sowohl des Menschen und der Tiere als auch von Pflanzen.

19. Verwendung des Verfahrens nach einem der Ansprüche 1 bis 15 zum Nachweis von Tumorerkrankungen sowohl von Menschen und Tieren als auch von Pflanzen.

20. Verwendung des Verfahrens nach einem der Ansprüche 1 bis 15 zur Kontrolle biologischer Proben.

21. Verwendung des Verfahrens nach einem der Ansprüche 1 bis 15 zur Zelltypisierung.

# FIG.1

1) Etape d'enrichissement

As1 →

a) AMPLIFICATION    D'ENRICHISSEMENT

As2

b) DILUTION

Ad4

2) Etape de détection

c) AMPLIFICATION    DE DETECTION

Ac3

Ad4

Ac3

d) FIXATION et
e) REVELATION

Ad4

Ac3    SUPPORT

# FIG. 2

$$Q = Q_0 \, (1+x)^n$$
avec $x = 0{,}70$

CINETIQUE D'AMPLIFICATION DE DETECTION

FIGURE 3